# EUROPEAN PATENT SPECIFICATION

(11) **EP 3 112 858 B1**
(45) Date of publication and mention of the grant of the patent: **23.05.2018**
(21) Application number: 15754822.3
(22) Date of filing: 23.02.2015
(51) Int. Cl.: G01N 30/88, G01N 30/02, G01N 30/60, G01N 33/02, B01D 15/08, B01D 15/30, B01J 20/24, B01J 20/281, B01J 20/291, B01J 20/34

(54) **PACKING MATERIAL FOR LIQUID CHROMATOGRAPHY AND COLUMN FOR LIQUID CHROMATOGRAPHY**
FÜLLSTOFF FÜR FLÜSSIGKEITSCHROMATOGRAPHIE UND FLÜSSIGKEITSCHROMATOGRAPHIESÄULE
PHASE POUR CHROMATOGRAPHIE LIQUIDE ET COLONNE DE CHROMATOGRAPHIE LIQUIDE

(30) Priority: 28.02.2014 JP 2014038331
(43) Date of publication of application: 04.01.2017
(73) Proprietor: Showa Denko K.K., Tokyo 105-8518 (JP)
(72) Inventor: NAKAJIMA, Naoya, Tokyo 105-8518 (JP); OKADA, Yoshiji, Tokyo 105-8518 (JP); KONDO, Hideyuki, Tokyo 105-8518 (JP)
(74) Representative: Strehl Schübel-Hopf & Partner
(86) International application number: PCT/JP2015/055031
(87) International publication number: WO 2015/129622

(56) References cited:
- CN-A- 101 121 118
- JP-A- H10 177 015
- JP-A- S60 232 254
- JP-A- 2013 075 954
- US-A1- 2008 169 240
- JULIO SÁNCHEZ ET AL: "Boron removal by liquid-phase polymer-based retention technique using poly(glycidyl methacrylate N-methyl D-glucamine)", JOURNAL OF APPLIED POLYMER SCIENCE, vol. 129, no. 3, 15 December 2012 (2012-12-15), pages 1541-1545, XP055408671, US ISSN: 0021-8995, DOI: 10.1002/app.38851

## Description

### Technical Field

The present invention relates to a packing material for liquid chromatography, which is excellent in durability, and a column for liquid chromatography, which is filled with the material.

### Background Art

Saccharides are contained in foods, etc. in large amounts, and analyses of them are extremely important in food industry, etc. For the analyses of them, liquid chromatography is widely used. In the analyses of saccharides by liquid chromatography, ligand exchange columns and hydrophilic interaction columns are mainly used.

In an analysis using a ligand exchange column, it is necessary to carry out analysis under high-temperature conditions in order to inhibit anomer separation. For example, in Japanese Patent Laid-Open Publication No. 1986-71354 (patent literature 1), a column using, as a packing material, an ion-exchange resin particle using silver and alkali (earth) metal ions as counter ions of a sulfonic acid group is disclosed. In this patent literature 1, separation is carried out at a column temperature of 60°C. At such a high temperature, however, a metal ion that is ionically bonded to the material is liable to be desorbed, and there is a problem of change in the material with time.

In the case of using a hydrophilic interaction column, there is a column using a packing material composed of a polyacrylamide resin (e.g., Japanese Patent Laid-Open Publication No. 2006-137944: patent literature 2). In order to inhibit anomer separation, however, high-temperature conditions are necessary similarly to the ligand exchange column.

CN 101121118A (patent literature 3) describes a hydrophilic packing material for separation and purification of polysaccharides, said packing material being produced by reacting polyvinyl alcohol resin microspheres with epichlorohydrin, followed by the reaction with e.g. diethylenetriamine.

On the other hand, when a column using a packing material to which an amino group has been bonded (said column being referred to as an "amino column" hereinafter) is used, anomer separation can be inhibited by alkalinity of the amino group, and high-temperature conditions are not necessary, so that amino columns are widely used for saccharide analysis (J.C. Linden, et al., J. Chromatogr. A 105 (1975), 125-133). However, most of amino columns use a silica gel as a base material, and a silica gel to which an amino group has been bonded has poor stability, and there is a problem in durability of the columns.

As a material to which an amino group can be bonded and which has chemical stability, there is a material using an organic polymer carrier. As a material in which an amino group has been bonded to an organic polymer carrier, a compound in which N-methyl-D-glucamine has been bonded to a polyacrylate-based resin or a styrene/divinylbenzene copolymer resin is used as an adsorbent adsorbing a metal or boron (J. Sanchez, et al., J. APPL POLY. SCI., 129 (2013), 1541-1545, J. Sanchez, et al., J. APPL POLY. SCI., 126 (2012), 1475-1483).

However, if a material using a polyacrylyte-based resin or a styrene/divinylbenzene copolymer resin is applied to an amino column, there are problems that the hydrophilicity of the copolymer resin is low and separation of saccharides is insufficient.

### Citation List

### Patent Literature

Patent literature 1: Japanese Patent Laid-Open Publication No. 1986-71354
Patent literature 2: Japanese Patent Laid-Open Publication No. 2006-137944
Patent literature 3: Chinese Patent Application No. 101121118A

### Non Patent Literature

Non patent literature 1: J. Sanchez, et al., J. APPL POLY. SCI., 129 (2013), 1541-1545
Non patent literature 2 : J. Sanchez, et al., J. APPL POLY. SCI., 126 (2012), 1475-1483

### Summary of Invention

### Technical Problem

It is an object of the present invention to provide a packing material suitable for an amino column which is excellent in durability and does not undergo changes with time even if analysis is carried out repeatedly.

### Solution to Problem

In order to achieve the above object, the present inventors have further studied. As a result, they have found that durability of an amino column can be improved by using a packing material which uses, as a base material, a hydrophilic resin comprising a polyvinyl alcohol resin and in which a specific amino group has been introduced, and they have accomplished the present invention.

That is to say, the present invention relates to the following matters.
[1] A packing material for liquid chromatography, wherein an amino group represented by the formula (1) is bonded through a spacer to a hydrophilic resin comprising a polyvinyl alcohol resin, 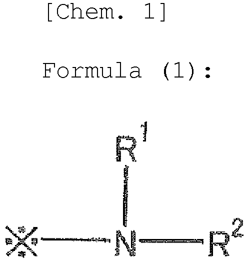 wherein R¹ represents a hydrogen atom or an alkyl group having 1 to 4 carbon atoms, R² represents an alkyl group having 1 to 6 carbon atoms and having one or more hydroxyl groups, and 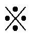 represents a bonding position to the spacer.
[2] The packing material for liquid chromatography of [1], wherein R¹ is a hydrogen atom or a methyl group.
[3] The packing material for liquid chromatography of [1] or [2], wherein R² has a structure represented by the formula (2): 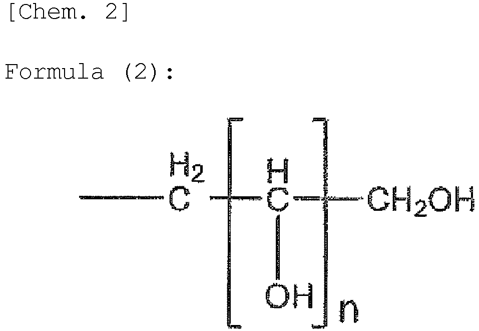 wherein n represents an integer of 0 to 4.
[4] The packing material for liquid chromatography of [1], wherein the amino group represented by the formula (1) is derived from any one amine of the group consisting of D-glucamine, N-methyl-D-glucamine, 1-amino-1-deoxy-D-mannitol, 1-amino-1-deoxy-D-galactitol, 1-amino-1-deoxy-D-iditol, 1-amino-1-deoxy-D-arabinitol, 1-amino-1-deoxy-D-xylitol, 4-amino-1,2,3-butanetriol, 3-amino-1,2-propanediol and 3-methylamino-1,2-propanediol.
[5] The packing material for liquid chromatography of [1], wherein the spacer is derived from a compound having a glycidyl group at an end.
[6] A column for liquid chromatography, using the packing material for liquid chromatography of any one of [1] to [5].
[7] A column for liquid chromatography for saccharide analysis, using the packing material for liquid chromatography of any one of [1] to [5].
[8] A column for hydrophilic interaction chromatography, using the packing material for liquid chromatography of any one of [1] to [5].

### Advantageous Effects of Invention

The packing material for liquid chromatography according to the present invention does not need high-temperature conditions in analysis of saccharides and exhibits excellent separation even at low temperatures such as 30 to 50°C. Further, the packing material can separate not only saccharides but also various hydrophilic compounds. Furthermore, the packing material has alkali resistance, and as compared with conventional amino columns, the packing material is excellent in reproducibility and durability.

### Brief Description of Drawings

[Fig. 1] Fig. 1 shows a chromatogram obtained by carrying out analysis of four kinds of saccharides of fructose, mannose, glucose and sucrose with regard to the packing material of Example 1.
[Fig. 2] Fig. 2 shows results of a stability test of the packing material of Example 1.
[Fig. 3] Fig. 3 shows a chromatogram obtained by carrying out analysis of four kinds of saccharides with regard to the packing material of Example 2.
[Fig. 4] Fig. 4 shows a chromatogram obtained by carrying out analysis of four kinds of saccharides with regard to the packing material of Example 3.
[Fig. 5] Fig. 5 shows a chromatogram obtained by carrying out analysis of four kinds of saccharides with regard to the packing material of Comparative Example 2.
[Fig. 6] Fig. 6 shows a chromatogram obtained by carrying out analysis of five kinds of saccharides of fructose, galactose, lactulose, epilactose and lactose with regard to the packing material of Example 1.
[Fig. 7] Fig. 7 shows a chromatogram obtained by changing the eluent and carrying out analysis of five kinds of saccharides in Analysis Example 6.

### Description of Embodiments

The packing material for liquid chromatography of the present invention is a particle in which an amino group represented by the formula (1) has been bonded to a hydrophilic resin through a spacer, 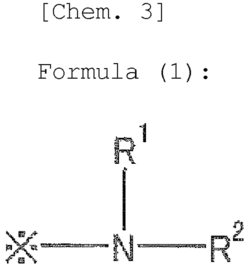 wherein R¹ represents a hydrogen atom or an alkyl group having 1 to 4 carbon atoms, R² represents an alkyl group having 1 to 6 carbon atoms and having one or more hydroxyl groups, and 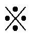 represents a bonding position to the spacer. R¹ is preferably a hydrogen atom or a methyl group.

R² is preferably a hydroxyalkyl group represented by the formula (2) : 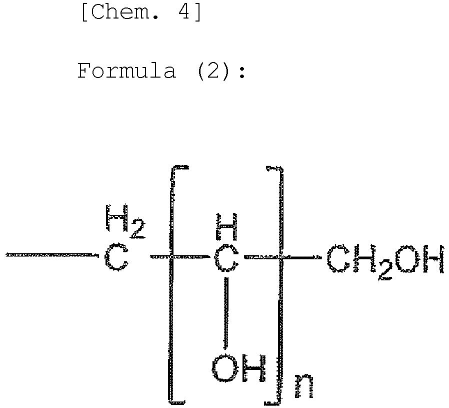 wherein n is an integer of 0 to 4. n is preferably 4.

The amino group represented by the formula (1) is preferably an amino group derived from any one of amines of the group consisting of D-glucamine, N-methyl-D-glucamine, 1-amino-1-deoxy-D-mannitol, 1-amino-1-deoxy-D-galactitol, 1-amino-1-deoxy-D-iditol, 1-amino-1-deoxy-D-arabinitol, 1-amino-1-deoxy-D-xylitol, 4-amino-1,2,3-butanetriol, 3-amino-1,2-propanediol and 3-methylamino-1,2-propanediol. One hydrogen bonded to nitrogen of any of these amines is desorbed, and the amine is bonded to a spacer. In the present invention, any of these amines has been bonded to a spacer, but it is also possible that the amino group is one obtained by bonding ammonia to a hydrophilic resin through a spacer and then bonding aldose, such as glucose, mannose, galactose or xylose, to a nitrogen atom constituting an amino group by the use of sodium cyanoborohydride. Of these, an amino group obtained by allowing N-methyl-D-glucamine to undergo reaction is particularly preferable from the viewpoints of separation performance, simplicity of reaction and ease of obtaining a reagent.

The packing material in which such an amino group has been bonded through a spacer does not need high-temperature conditions in analysis of saccharides, and even at low temperatures such as 30 to 50 °C, the packing material exhibits excellent separation, has alkali resistance and is excellent in reproducibility and durability.

There is no specific limitation on the size and the shape of the hydrophilic resin that constitutes the packing material in the present invention. However, when filling of a column with the packing material of the present invention is taken into consideration, the shape of the resin is preferably a spherical shape having a diameter of 1 to 30 µm. As the material, a crosslinked or uncrosslinked resin is preferable. A resin having an alcoholic hydroxyl group is particularly preferably used.

The resin for use in the present invention is preferably a polyvinyl alcohol-based resin wherein an ester group of a crosslinked copolymer of a carboxylic acid vinyl ester and a crosslinkable monomer has been converted into an alcoholic hydroxyl group by saponification or ester exchange reaction. Examples of the carboxylic acid vinyl esters include vinyl acetate, vinyl propionate, vinyl butyrate, vinyl valerate and vinyl pivalate. These may be used singly or may be used in combination of two or more kinds. Of these, vinyl acetate and vinyl propionate are preferable because polymerization of monomers and saponification of ester group are easy. As the crosslinkable monomers, crosslinkable monomers having a triazine ring are preferable, and of these, triallyl isocyanurate is particularly preferable.

The above resins do not have a structure having low resistance to alkalinity, such as an ester structure or an amide structure. On that account, the packing material of the present invention is stable even under alkalinity. Hence, there is an advantage that the packing material can be preferably used even under alkaline analytical conditions.

In addition to the above resin, other resins may be contained within limits not detrimental to the object of the present invention. For example, a styrene/divinylbenzene-based crosslinked polymer, a methacrylate-based crosslinked polymer, a resin in which a long-chain acyl group is chemically bonded to a hydroxyl group-containing methacrylate-based crosslinked polymer particle, etc. are also employable. The content of these other resins is not more than 10% by mass, preferably not more than 5%bymass, based on the resin quantity. The hydrophilic resin is more preferably one composed of a polyvinyl alcohol resin from the viewpoints of durability and resistance to alkalinity.

The amount of the amino group is preferably in the range of 0.1 to 0.7 meq/g, more preferably 0.15 to 0.5 meq/g, based on the resin weight. When the amount of the amino group is in this range, analysis of saccharides becomes possible even under low-temperature conditions.

The spacer in the present invention refers to a site of chemical bonding used for adjusting a distance between the base material (hydrophilic resin) and the amino group. The spacer is used for imparting functions to inhibit interference between the amino group of the formula (1) and the base material and to inhibit peak diffusion. As a compound used for introducing a spacer into the base material, namely, a so-called spacer compound, a compound having a glycidyl group can be mentioned. Examples of such compounds include epichlorohydrin, 1,4-butanediol diglycidyl ether, ethylene glycol diglycidyl ether and glycerol diglycidyl ether. A terminal halogen group or one terminal glycidyl group of these spacer compounds reacts with a hydroxyl group of the hydrophilic resin.

The amount of a glycidyl group introduced is preferably in the range of 0.2 to 0.8 mol/g, more preferably 0.4 to 0.6 mol/g. When the amount thereof is in this range, a preferred amount of an amino group can be introduced.

Introduction of a glycidyl group is carried out by adding the above reagent 0.1 to 10 times as much as the gel in the absence of a solvent or in the presence of a solvent and homogeneously stirring them.

The reaction of the polymer in which a glycidyl group has been introduced with the aforesaid amine for introducing an amino group represented by the formula (1) can be carried out in the following manner. First, the polymer in which a glycidyl group has been introduced is dispersed in a solvent, such as water, dimethyl sulfoxide or N,N-dimethylformamide. As the solvent used herein, water is most preferable from the viewpoints of ease of handling, solubility of amine used, etc. The amine is preferably added in an amount of not less than 2 mmol based on 1 g of the polymer in which a glycidyl group has been introduced, and the amine is more preferably added in an amount of 10 mmol to 20 mmol from the viewpoints of reaction efficiency and reproducibility. The reaction temperature is preferably 30 to 40 °C from the viewpoints of reproducibility and reaction efficiency. The reaction time is preferably 12 hours to 24 hours from the viewpoints of reaction quantity and efficiency.

The particle diameter of the packing material for liquid chromatography according to the present invention is 1 to 30 µm, preferably 2 to 20 µm, more preferably 2 to 10 µm, in terms of volume-average particle diameter. If the volume-average particle diameter is less than 1 µm, a rise in pressure of a column is large, and filling sometimes becomes extremely difficult. On the other hand, if the volume-average particle diameter exceeds 30 µm, the number of theoretical plates of a column is decreased, so that such a particle diameter is undesirable. The volume-average particle diameter in the present application is measured in the following manner using a Coulter counter method. That is to say, using Multisizer 4 (manufactured by Beckman Coulter Inc.) as a measuring device, 25 mL of Isoton (diluent liquid) is added to 0.2 g of a packing material sample, then ultrasonic waves are applied for 3 minutes to disperse the sample, and thereafter, a volume-average particle diameter of about 1000 measurement particles is measured. The volume-average particle diameter can be adjusted to be within a preferred range by air classification, classification by sieving, classification using sedimentation, etc.

Filling of a column with the packing material for liquid chromatography of the present invention can be carried out in accordance with a publicly known filling method, such as slurry method. By using, as an eluent, a mixed solvent of acetonitrile and water or a buffer solution containing ammonium formate, ammonium acetate or the like in the solvent, the resulting column for liquid chromatography can well separate saccharides or other hydrophilic compounds.

Examples of the separation object substances in the present invention include environmental pollutants, dioxins, environmental hormones, agricultural chemicals, surface active agents, biological toxins, natural drugs, natural dyes, natural perfumes and natural seasonings.

The present invention can provide a packing material suitable for an amino column which is excellent in durability and does not undergo changes with time even if analysis is carried out repeatedly. In particular, the packing material can carry out separation even at low temperatures, and therefore, the packing material is applicable also to uses such as analysis of saccharides having unstable properties at high temperatures.

### Examples

The present invention is described in more detail with reference to the following examples, but it should be construed that the present invention is in no way limited to those examples.

### [Example 1]

### Polyvinyl alcohol gel (base material)

In a 5L three-neck flask equipped with a reflux condenser, a homogeneous mixed liquid composed of 100 g of vinyl acetate, 150 g of triallyl isocyanurate, 100 g of butyl acetate, 25 g of n-decane and 5 g of 2,2-azobisisobutyronitrile, and 1200 mL of water in which 12 g of polyvinyl alcohol and 18 g of disodium hydrogenphosphate had been dissolved were placed, and they were stirred for 10 minutes. Subsequently, while stirring the contents in the flask in a stream of nitrogen, polymerization was carried out at 60 °C for 16 hours to obtain a particulate polymer. The polymer was subjected to filtration, washing and acetone extraction, and then dried. Subsequently, in a 5L three-neck flask equipped with a reflux condenser, a nitrogen feed pipe and a stirrer, the polymer was placed together with 3 L of a 10% caustic soda aqueous solution, and the contents in the flask were stirred at 15°C for 20 hours in a stream of nitrogen to carry out saponification of the polymer. Thereafter, the resulting polymer was filtered, washed with water and further dried. The polyvinyl alcohol polymer obtained by saponification had a hydroxyl group density of 2.1 meq/g.

### Packing material for saccharide analysis and hydrophilic interaction chromatography

In a separable flask, 10 g of the dry polyvinyl alcohol polymer, 45 g of epichlorohydrin, 100 mL of dimethyl sulfoxide and 5 mL of a 30% caustic soda aqueous solution were placed, and they were stirred at 30 °C for 20 hours to introduce a glycidyl group into the polymer. The polymer after introduction was washed with dimethyl sulfoxide, water and methanol, and then dried.

In a separable flask, 4 g of the polymer in which a glycidyl group had been introduced, 10 g of N-methyl-D-glucamine and 40 mL of water were placed, and they were stirred at 30°C for 20 hours to prepare a packing material for saccharide analysis and hydrophilic interaction chromatography, in said packing material an amino group having been introduced. The packing material was washed with 0.5N hydrochloric acid and a 0.5N caustic soda aqueous solution while interposing washing with water between those washing operations. The packing material was further washed with methanol and then dried. The packing material obtained as above was dispersed in a 0.5N potassium chloride aqueous solution, and titration of the amino group with 0.1M hydrochloric acid was carried out to measure an amino group density of the packing material. As a result, the amino group density was 0.44 meq/g. The volume-average particle diameter of the packing material was 5 µm.

### [Example 2]

In a separable flask, 2.0 g of the polymer in which a glycidyl group had been introduced, said polymer having been prepared in the same manner as in Example 1, 1.0 mL of 25% aqueous ammonia and 19 mL of water were placed, and they were stirred at 30°C for 20 hours to introduce an amino group. After the polymer was filtered, the polymer was washed with 0.5N hydrochloric acid and a 0.5N caustic soda aqueous solution while interposing washing with water between those washing operations. The polymer was further washed with methanol and then dried.

In a separable flask, 1.8 g of the polymer having an amino group, 1.9 g of D-mannose, 0.2 g of sodium cyanoborohydride and 12 mL of a 0.2M dipotassium hydrogenphosphate aqueous solution were placed, and they were stirred at 60 °C for 20 hours to prepare an amino group in which aldose had been bonded to a nitrogen atom, whereby a packing material for saccharide analysis and hydrophilic interaction liquid chromatography was prepared. The packing material was washed with 1.0N sulfuric acid and a 1.0N caustic soda aqueous solution while interposing washing with water between those washing operations, and then, the packing material was dried.

The amino group density of the resulting polymer having an amino group was measured in the same manner as in Example 1, and as a result, the amino group density was 0.26 meq/g.

### [Example 3]

A packing material for liquid chromatography was prepared under the same conditions as in Example 1, except that 100 g of 1, 4-butenediol diglycidyl ether was used instead of 45 g of epichlorohydrin. The amino group density of the packing material was measured in the same manner as in Example 1, and as a result, the amino group density was 0.17 meq/g.

### [Analysis Example 1]

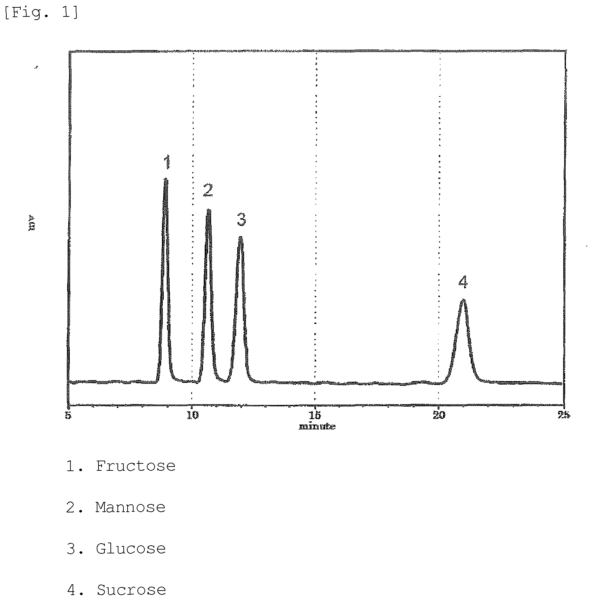

A SUS column having an inner diameter of 4.6 mm and a length of 150 mm was filled with the packing material obtained in Example 1 to prepare a column for saccharide analysis and hydrophilic interaction chromatography. Using this column, analysis was carried out under the conditions of an eluent of acetonitrile/water=85/15, a flow rate of 0.6 mL/min and a column temperature of 40°C. As an analysis sample, a solution obtained by dissolving four kinds of saccharides, namely, fructose, mannose, glucose and sucrose, in a mixed liquid of acetonitrile/water=5/3 in such a manner that each saccharide concentration became 5 mg/mL was used. A chromatogram obtained by using a differential refractive index detector is shown in Fig. 1. Four kinds of the saccharides are well separated from one another.

### [Analysis Example 2]

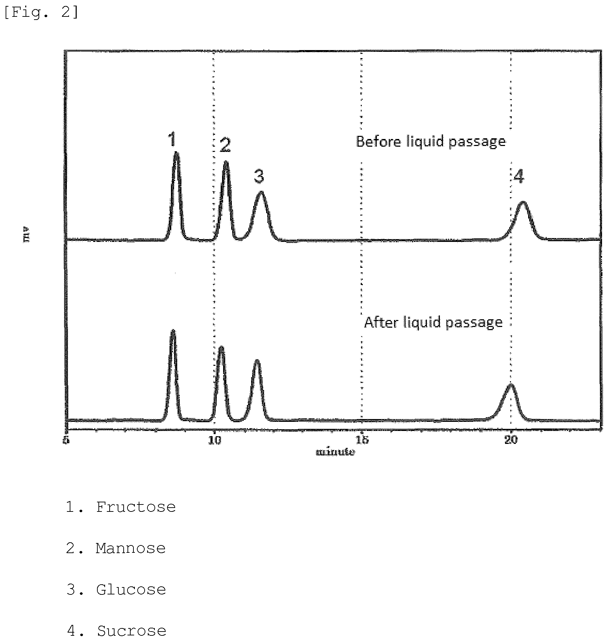

A stability test of the above column was carried out. An eluent of a 0.05N sodium hydroxide aqueous solution was continuously passed through the column for 80 hours under the conditions of a flow rate of 0.6 mL/min and a column temperature of 30 °C to confirm retention times of saccharides before and after the liquid passage and the number of theoretical plates. The results obtained by this test are shown in Fig. 2. In the case of the column using the packing material of the present invention, changes in retention time, etc. did not occur even though the eluent was continuously passed.

### [Analysis Example 3]

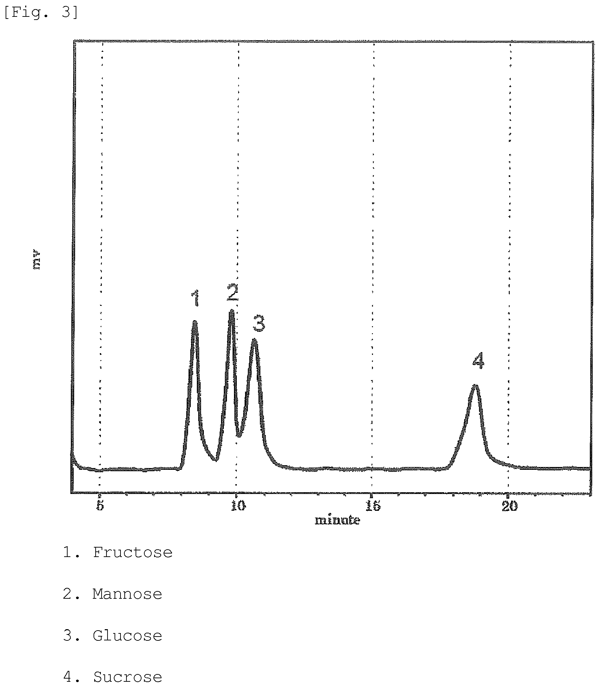

Using the packing material obtained in Example 2, the same analysis as in Analysis Example 1 was carried out. The results are shown in Fig. 3. Four kinds of the saccharides are well separated from one another.

### [Analysis Example 4]

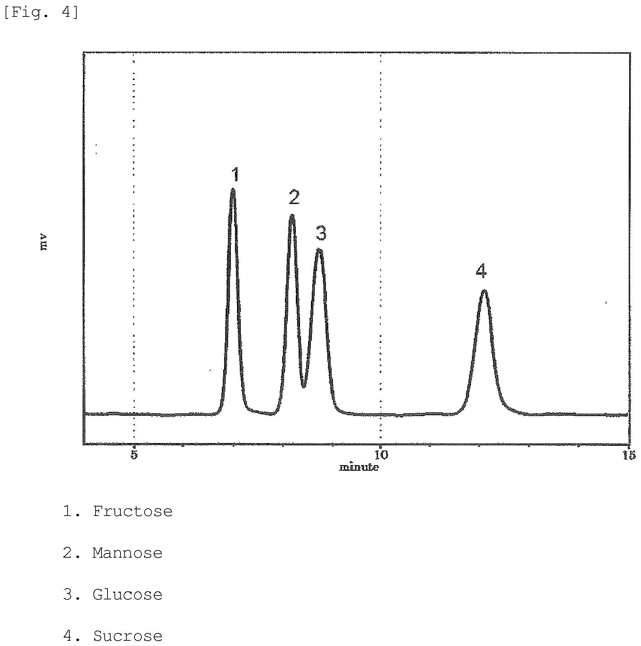

Using the packing material obtained in Example 3, the same analysis as in Analysis Example 1 was carried out. The results are shown in Fig. 4. Four kinds of the saccharides are well separated from one another.

### [Comparative Example 1]

By the use of a column using a packing material containing a silica gel as a base material and having an amino group introduced therein, the aforesaid stability test was carried out. As a result, after 17 hours from the beginning of liquid passage, clogging of the column took place to make liquid passage impossible.

### [Comparative Example 2]

In a separable flask, 2. 8 g of an acrylate resin in which glycidyl methacrylate had been polymerized, 6.5 g of N-methyl-D-glucamine and 28 mL of water were placed, and they were stirred at 30 °C for 20 hours to prepare a packing material containing an acrylate resin as a base material. After the polymer was filtered, the packing material was washed with 0.5N hydrochloric acid and a 0.5N caustic soda aqueous solution while interposing washing with water between those washing operations.

### [Analysis Example 5]

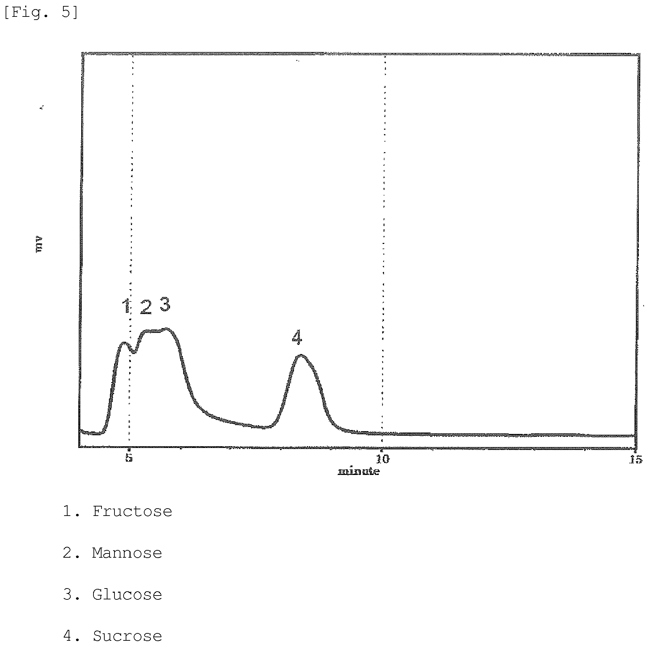

A column was filled with the packing material obtained in Comparative Example 2 in the same manner as in Analysis Example 1, and analysis of saccharides was carried out. The results are shown in Fig. 5. It can be seen that the retention time is short and the saccharides are not separated from one another.

### [Analysis Example 6]

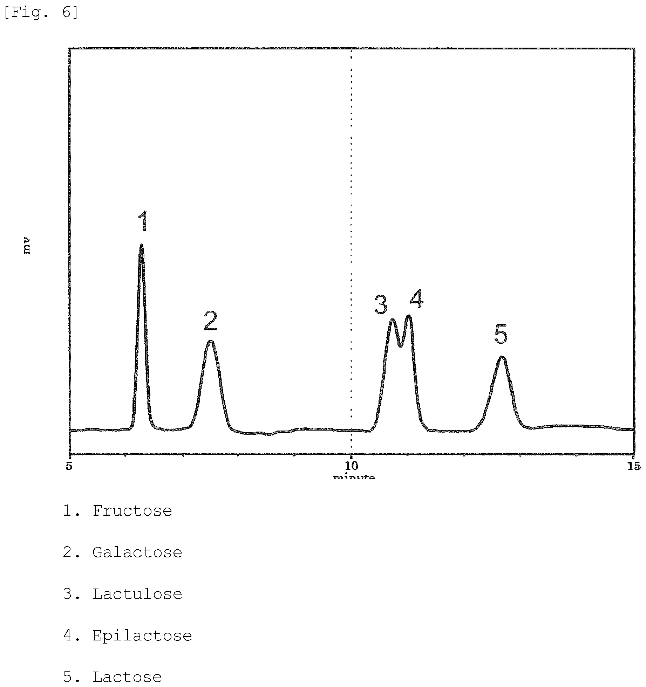

A SUS column having an inner diameter of 4.6 mm and a length of 250 mm was filled with the packing material obtained in Example 1 to prepare a column for saccharide analysis and hydrophilic interaction chromatography. Using this column, analysis of five kinds of saccharides, namely, fructose, galactose, lactulose, epilactose and lactose, was carried out. The analytical conditions were a flow rate of 1.0 mL/min and a column temperature of 40 °C. As a detector, a differential refractive index detector was used. A chromatogram obtained when a mixed liquid of acetonitrile/water=80/20 was used as an eluent is shown in Fig. 6.

### [Analysis Example 7]

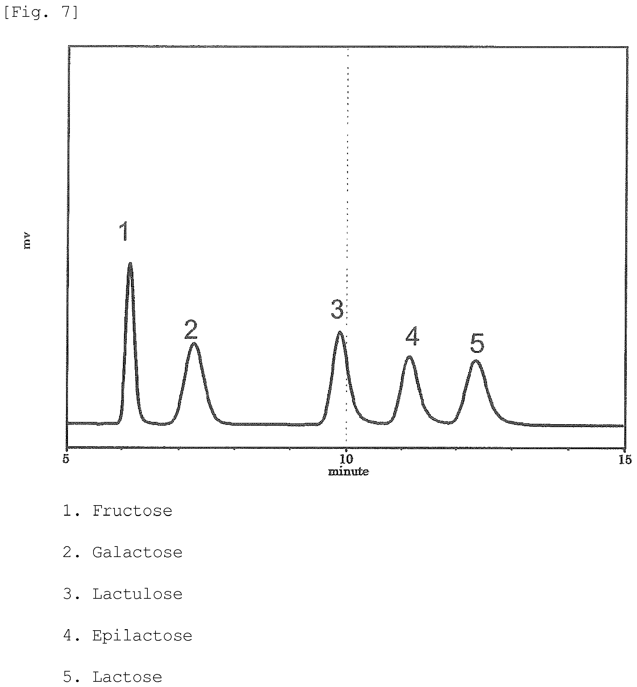

In Fig. 6 of Analysis Example 6, peaks of lactulose and epilactose partially overlap. Then, using the same column, the same sample was analyzed by changing the eluent to a mixed liquid of acetonitrile/methanol/water=75/20/5. The resulting chromatogram is shown in Fig. 7. Peaks of lactulose and epilactose, which overlap in Fig. 6, are completely separated from each other. Therefore, five kinds of the saccharides are well separated from one another.

## Claims

1. A packing material for liquid chromatography, wherein an amino group represented by the formula (1) is bonded through a spacer to a hydrophilic resin comprising a polyvinyl alcohol resin, 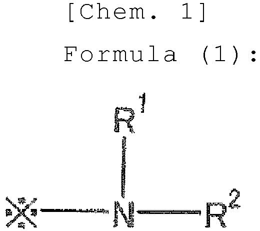 wherein R¹ represents a hydrogen atom or an alkyl group having 1 to 4 carbon atoms, R² represents an alkyl group having 1 to 6 carbon atoms and having one or more hydroxyl groups, and 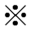 represents a bonding position to the spacer.

2. The packing material for liquid chromatography as claimed in claim 1, wherein R¹ is a hydrogen atom or a methyl group.

3. The packing material for liquid chromatography as claimed in claim 1 or 2, wherein R² has a structure represented by the formula (2): 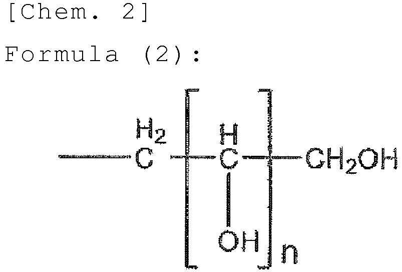 wherein n represents an integer of 0 to 4.

4. The packing material for liquid chromatography as claimed in claim 1, wherein the amino group represented by the formula (1) is derived from any one amine of the group consisting of D-glucamine, N-methyl-D-glucamine, 1-amino-1-deoxy-D-mannitol, 1-amino-1-deoxy-D-galactitol, 1-amino-1-deoxy-D-iditol, 1-amino-1-deoxy-D-arabinitol, 1-amino-1-deoxy-D-xylitol, 4-amino-1,2,3-butanetriol, 3-amino-1,2-propanediol and 3-methylamino-1,2-propanediol.

5. The packing material for liquid chromatography as claimed in claim 1, wherein the spacer is derived from a compound having a glycidyl group at an end.

6. Use of the packing material for liquid chromatography as claimed in claims 1 to 5, for a column for liquid chromatography.

7. Use of the packing material for liquid chromatography as claimed in claims 1 to 5, for a column for liquid chromatography for saccharide analysis.

8. Use of the packing material for liquid chromatography as claimed in claims 1 to 5, for a column for hydrophilic interaction chromatography.

## Patentansprüche

1. Packmaterial für Flüssigchromatographie, wobei eine Aminogruppe der folgenden Formel (1) über einen Spacer an ein hydrophiles Harz, umfassend ein Polyvinylalkoholharz, gebunden ist, 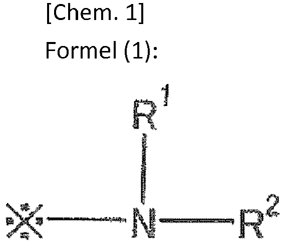 worin R¹ ein Wasserstoffatom oder eine Alkylgruppe mit 1 bis 4 Kohlenstoffatomen darstellt, R² eine Alkylgruppe mit 1 bis 6 Kohlenstoffatomen darstellt und eine oder mehrere Hydroxylgruppen aufweist und 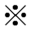 eine Bindungsposition an den Spacer darstellt.

2. Packmaterial für Flüssigchromatographie nach Anspruch 1, worin R¹ ein Wasserstoffatom oder eine Methylgruppe ist.

3. Packmaterial für Flüssigchromatographie nach Anspruch 1 oder 2, worin R² eine durch die Formel (2) dargestellte Struktur aufweist: 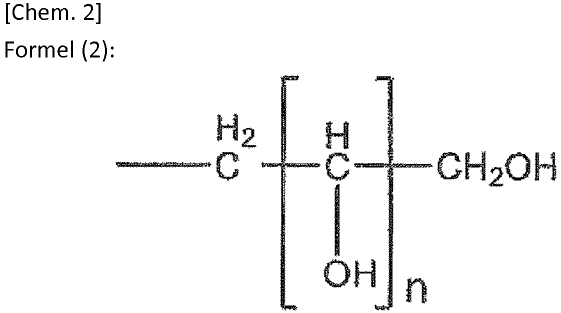 worin n eine ganze Zahl von 0 bis 4 darstellt.

4. Packmaterial für Flüssigchromatographie nach Anspruch 1, worin die durch die Formel (1) dargestellte Aminogruppe von einem Amin aus der Gruppe abgeleitet ist, die aus D-Glucamin, N-Methyl-D-glucamin, 1-Amino-1-deoxy-D-mannitol, 1-Amino-1-deoxy-D-galactitol, 1-Amino-1-deoxy-D-iditol, 1-Amino-1-deoxy-D-arabinitol, 1-Amino-1-deoxy-D-xylitol, 4-Amino-1,2,3-butantriol, 3-Amino-1,2-propandiol und 3-Methylamino-1,2-propandiol besteht.

5. Packmaterial für Flüssigchromatographie nach Anspruch 1, worin der Spacer von einer Gruppe abgeleitet ist, die an einem Ende eine Gycidylgruppe aufweist.

6. Flüssigchromatographiesäule, bei welcher das Packmaterial für Flüssigchromatographie nach einem der Ansprüche 1 bis 5 verwendet wird.

7. Flüssigchromatographiesäule zur Saccharidanalyse, bei welcher das Packmaterial für Flüssigchromatographie nach einem der Ansprüche 1 bis 5 verwendet wird.

8. Säule zur hydrophilen Wechselwirkungschromatographie, bei welcher das Packmaterial für Flüssigchromatographie nach einem der Ansprüche 1 bis 5 verwendet wird.

## Revendications

1. Phase de chromatographie liquide, dans laquelle un groupe amino représenté par la formule (1) se lie par l'intermédiaire d'un espaceur à une résine hydrophile comprenant une résine d'alcool polyvinylique, 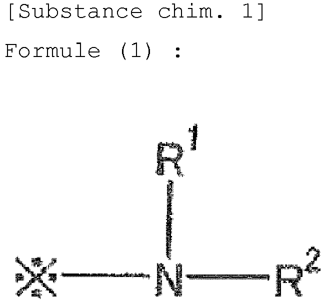 dans laquelle R¹ représente un atome d'hydrogène ou un groupe alkyle comportant de 1 à 4 atomes de carbone, R² représente un groupe alkyle comportant de 1 à 6 atomes de carbone et comportant un ou plusieurs groupes hydroxyles, et 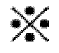 représente une position de liaison avec l'espaceur.

2. Phase de chromatographie liquide selon la revendication 1, dans laquelle R¹ est un atome d'hydrogène ou un groupe méthyle.

3. Phase de chromatographie liquide selon la revendication 1 ou 2, dans laquelle R² a une structure représentée par la formule (2) : 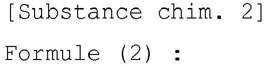 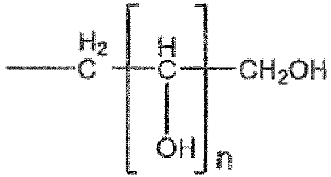 dans laquelle n représente un nombre entier de 0 à 4.

4. Phase de chromatographie liquide selon la revendication 1, dans laquelle le groupe amino représenté par la formule (1) est dérivé d'une amine quelconque du groupe constitué de la D-glucamine, de la N-méthyl-D-glucamine, du 1-amino-1-désoxy-D-mannitol, du 1-amino-1-désoxy-D-galactitol, du 1-amino-1-désoxy-D-iditol, du 1-amino-1-désoxy-D-arabinitol, du 1-amino-1-désoxy-D-xylitol, du 4-amino-1,2,3-butanetriol, du 3-amino-1,2-propanediol et du 3-méthylamino-1,2-propanediol.

5. Phase de chromatographie liquide selon la revendication 1, dans laquelle l'espaceur est dérivé d'un composé comportant un groupe glycidyle à une extrémité.

6. Utilisation de la phase de chromatographie liquide selon les revendications 1 à 5, pour une colonne de chromatographie liquide.

7. Utilisation de la phase de chromatographie liquide selon les revendications 1 à 5, pour une colonne de chromatographie liquide pour l'analyse de saccharides.

8. Utilisation de la phase de chromatographie liquide selon les revendications 1 à 5, pour une colonne de chromatographie d'interaction hydrophile.
